# EUROPEAN PATENT APPLICATION

(11) **EP 3 769 658 A1**
(43) Date of publication of application: **27.01.2021**
(21) Application number: 19187833.9
(22) Date of filing: 23.07.2019
(51) Int. Cl.: A61B 1/00, A61B 1/05, A61B 1/227

(54) **A SCOPE**

(71) Applicant: National University of Ireland Galway, Galway (IE)
(72) Inventor: O'Callaghan, Rory, Shercock, Co. Cavan (IE); Keogh, Ivan, Renmore, Co. Galway (IE); Gallagher, Martin, Swinford, Co. Mayo (IE); McGloughlin, Elizabeth, Ballyclough, Co. Limerick (IE); Walsh, Christina, Roscam, Co. Galway (IE)
(74) Representative: Purdylucey Intellectual Property

(57) **Abstract**

A scope (1) for examining or surgically treating ears comprising a probe (2); at least one visualiser (3) on the probe having an optical configuration (7), and a light source (42) wherein the visualiser (3) is articulatable about a visualiser articulation axis (3a) by an orienting mechanism (4) for optimal viewing of the ear.

## Description

### Introduction

This invention relates to a scope and more particularly to a medical scope such as an otoscope or an endoscope for examining and surgically treating ears.

### Background of the Invention

Medical devices of various types are employed to examine and perform surgery on ears. For example, endoscopes and otoscopes (hereinafter referred to collectively as scopes) are instruments which are held against the eye to view and magnify the subject. These instruments have evolved with the addition of a camera so that the subject is viewed using a computer monitor or a video display.

Recurrent acute otitis media, otitis media with effusion, chronic secretory / suppurative otitis media continues to impact quality of life for millions of patients world-wide. Hearing loss, complications of inflammation and complications of treatment are daily challenges and, as a result, patients with these conditions (children and adults) frequently require invasive ear surgery. As ears are wrapped in dense bone, surgical access and visualization are essentials for safe ear surgery.

In traditional ear surgery, microscopic visualization is employed with a dynamic bi-manual surgical technique. However, a major disadvantage of this approach is the narrow field of view looking down into the ear canal which can lead to poorer visualization of disease in difficult to access areas in the middle ear, such as the sinus tympani and facial recess.

In order to improve the field of view, it is often necessary to expose the middle ear and attic area by performing a mastoidectomy. However, mastoidectomy procedures are associated with increased operating times, more serious complications, longer hospital stays and protracted recovery for patients.

In addition, trans canal surgery is generally performed through a speculum by looking through the microscope and using specialized instruments resulting in a narrow field of view.

Moreover, due to the large size of the microscopic equipment employed in ear surgeries, negative ergonomic issues arise - the surgeon is forced into an extended position due to the size of the operating microscope which can compromise the dexterity required of the surgeon during surgical procedures.

In addition, when using a traditional analogue otoscopes or endoscopes, the human eye is effectively the image sensor utilizing the magnification provided by the otoscope/endoscope optics. Accordingly, when the otoscope/endoscope is reoriented or rotated in the ear canal the image observed remains oriented with respect to the user (independent of the otoscope/endoscope). However, with a video otoscope or endoscope the image sensor is located on the scope. Accordingly, when the scope is reoriented or rotated (perhaps for the comfort of the patient) the image observed also reorients or rotates which can result in disorientation of the user.

Moreover, in use, tissue can frequently adhere to scopes which can obscure vision with the result that the scope must regularly be removed from a subject's ear during surgery for cleaning which interrupts and prolongs the surgical procedure.

Overall, difficulties are encountered by surgeons visualising and focusing on the internal structures of the ear during surgery using known scopes in which the ability to accurately focus the scope and orient the scope for optimal viewing is absent.

An object of the invention is to overcome at least some of the problems of the prior art.

### Summary of the Invention

According to a first aspect of the invention there is provided a scope for examining or surgically treating a part of the body comprising:
a probe;
at least one visualiser on the probe having an optical configuration, and
a light source,
wherein the visualiser is articulatable relative to the probe by an orienting mechanism for optimal viewing of the body part.

In one embodiment, the part of the body is a difficult to access part of the body, such as the internal part of the ear, nose, throat, or another body orifice.

In one embodiment, the orienting mechanism is configured to tilt or rotate the visualiser relative to the probe.

In one embodiment, the visualiser is articulatable about a visualiser articulation axis by an orienting mechanism for optimal viewing of the ear.

Preferably, the visualiser is rotatable about the visualiser articulation axis. More preferably, the visualiser has a uniform profile about its articulation axis.

Most preferably, the visualiser is substantially spherical. In one embodiment, the probe comprises a socket configured for receipt of the substantially spherical visualiser for rotation of the visualiser about multiple axes. In one embodiment, the orienting mechanism comprises a roller in contact with the substantially spherical visualiser, whereby rotation of the roller effects rotation of the substantially spherical visualiser. In one embodiment, the orientating mechanism comprises a first roller in contact with the substantially spherical visualiser and configured for rotation about a first axis of rotation, and a second roller in contact with the substantially spherical visualiser and configured for rotation about a second axis of rotation, wherein the first and second axes of rotation are optionally orthogonal to each other.

Suitably, the visualiser comprises an image sensor or a camera.

In one embodiment, the probe comprises a hinge.

Suitably, the orienting mechanism is a belt- or band- driven orienting mechanism, or a fluidic (i.e. pneumatic or hydraulic) orienting mechanism In one embodiment, the orienting mechanism comprises a wheel or cog operatively coupled to the visualiser. In one embodiment, the orienting mechanism comprises a magnetic force generator configured to act on the visualiser. In one embodiment, the scope is configured for manual actuation of the orienting mechanism. In one embodiment, the orienting mechanism comprises a motor configured to drive the articulation of the visualiser.

In a preferred embodiment, the scope further comprises a self-cleaning module for cleaning detritus from the visualiser. Preferably, the self-cleaning module comprises a cleaning blade and/or a soft cleaning material. In one embodiment, the visualiser is configured for movement relative to the self-cleaning module to clean the visualiser. In one embodiment, the self-cleaning module comprises a sheath configured for movement from a non-deployed position to a deployed position in which the sheath closes over the lens of the visualiser wiping the lens. In this embodiment, the visualiser may be configured for movement to a cleaning position when the sheath is deployed, for example retracted into the sheath and/or tilted to one side.

Preferably, the optical configuration comprises lens elements configured to allow for variable focusing and refocusing.

Suitably, the lens elements comprise an internal lens within the visualiser.

In a preferred embodiment, the lens elements comprise an external curved lens on the visualiser.

The invention also extends to a scope further comprising a stabilizer for supporting the scope. Preferably, the stabilizer comprises a speculum.

In a particularly preferred embodiment of the invention, the scope comprises an endoscope or an otoscope.

In another embodiment, the invention relates to a scope for examining or surgically treating a part of the body (for example a difficult to access part of the body such as the ear, nose or throat) comprising:
a probe;
at least one visualiser on the probe,
an optical configuration including image sensors, and
a light source
wherein the optical configuration is stacked in the probe to save space and reduce the profile of the probe. Preferably, the image sensors comprise image sensor boards.

Suitably, the optical configuration comprises first and second stacked fixed image sensors. Preferably, the optical configuration comprises corresponding first and second lens elements directing light to the image sensors. Advantageously, the optical configuration comprises a slidably adjustable front lens.

In one embodiment, the optical configuration comprises aspherical lenses to bypass the first fixed image sensor. Preferably the aspherical lenses comprise crescent profile lenses.

In a further embodiment, the invention relates to a scope for examining or surgically treating a part of the body (for example a difficult to access part of the body such as the ear, nose or throat) comprising:
a probe;
at least one visualiser on the probe having an optical configuration, and
a light source
wherein the visualiser is articulatable about a visualiser articulation axis by an orienting mechanism for optimal viewing of the ear and the articulatable visualiser is a tiltable camera

Preferably, the orienting mechanism comprises a directional lever.

Alternatively, the orienting mechanism comprises a directional wheel and the probe is slidable to adjust the depth of the probe. In a still further embodiment, the orienting mechanism comprises a slider either alone or in combination with the directional wheel and/or directional lever.

Preferably, the scope further comprises a stabilizer for supporting the tiltable visualiser in or on the stabilizer, the stabilizer being integral with the tiltable visualiser and being configured to stabilize the scope in an ear canal. More preferably, the probe is mountable on the stabilizer at a probe mounting.

Suitably, the stabilizer comprises a speculum holder. In a preferred embodiment, the stabilizer comprises a detachable or integrated speculum. Advantageously, light from the light source is transmissible through the speculum.

In a still further embodiment, the invention relates to a scope for examining or surgically treating a part of the body (for example a difficult to access part of the body such as the ear, nose or throat) comprising:
a probe;
at least one visualiser on the probe,
an optical configuration, and
a light source
wherein the optical configuration includes an angled prism adjacent the visualiser to assist in unobtrusive visualisation. Preferably, the visualiser comprises a camera.

In a further embodiment, the invention relates to a scope for examining or surgically treating a part of the body (for example a difficult to access part of the body such as the ear, nose or throat) comprising:
a probe;
at least one visualiser on the probe having an optical configuration, and
a light source
wherein the visualiser is articulatable about a visualiser articulation axis by an orienting mechanism for optimal viewing of the part of the body, the articulatable visualiser is a tiltable camera and the orienting mechanism comprises a living hinge on the probe.

Preferably, the living hinge comprises an external living hinge. Alternatively, the living hinge comprises an internal living hinge. Suitably, the living hinge comprises a double living hinge. Advantageously, the double living hinge comprises a spring-operated double living hinge.

Optionally, the optical configuration includes an angled prism to assist in unobtrusive visualisation.

In a still further embodiment, the invention relates to a scope for examining or surgically treating a part of the body (for example a difficult to access part of the body such as the ear, nose or throat) comprising:
a probe;
at least one visualiser on the probe,
an optical configuration, and
a light source
wherein the optical configuration includes lens elements which are adjustable to focus images. Suitably, the optical configuration comprises combinations of an adjustable lens and sensor element, a fixed front element, and adjustable sensor, a rod lens, an adjustable front lens and an angled front lens.

In another embodiment, the invention relates to a scope for examining or surgically treating a part of the body (for example a difficult to access part of the body such as the ear, nose or throat) comprising:
a probe;
at least one visualiser on the probe,
an optical configuration, and
a light source
wherein the optical configuration includes mechanically coupled lens elements.

The invention also relates to a scope for examining or surgically treating a part of the body (for example a difficult to access part of the body such as the ear, nose or throat) comprising:
a probe;
at least one visualiser on the probe,
an optical configuration, and
a light source
wherein the optical configuration is a staged focus optical configuration. Preferably, the staged focus optical configuration comprises a solenoid and spring-operated staged focus optical configuration. Alternatively, the staged focus optical configuration comprises a ratchet mechanism or a friction controlled mechanism.

The articulatable visualiser and in particular the rotatable visualiser is particularly beneficial in minimal invasive surgery as the eyeball camera can point in different directions including straight ahead (0°) and rotated to different angles. In ear surgery, this allows a surgeon to see around the corner of the ear canal or up into the epitympanum / attic in the middle ear.

The self-cleaning module of the scope of the invention removes blood and other debris that can adhere to the front lens, so that the scope or probe do not need to be removed during surgery for cleaning thus avoiding disruption or delay during surgery.

The optical configuration of the scope of the invention is configured to allow for variable focusing and refocusing as required e.g. when the front lens is positioned above a surgical site to observe operation of tools or, at other times, when the front lens is pushed up close to observe tissue structure.

The scope of the invention, and in particular, the probe and articulatable visualiser in the form of the eyeball camera can be easily disassembled for cleaning and re-use.

### Brief Description of the Drawings

The invention will now be described, by way of example only, with reference to the accompanying drawings in which:
Figure 1 (a) is a perspective view from below and one side of a first embodiment of a scope of the invention made up of a probe and a uniform articulatable visualiser on the probe in the form of a rotatable camera eyeball operable by a visualiser orienting mechanism;
Figure 1(b) is an enlarged perspective view from below and one side of the eyeball of Figure 1(a);
Figure 2 is an exploded perspective view from below of the scope of Figures 1(a) and 1(b);
Figure 3 is a side elevation of the scope provided with a belt-driven visualiser orienting mechanism with the direction of rotation of the belt and eyeball indicated by arrows;
Figure 4 is a side elevation of the scope provided with a hydraulic visualiser orienting mechanism with the direction of rotation of the eyeball and hydraulic fluid indicated by arrows;
Figure 5 is a side elevation of the scope provided with a centrifugal or pelton wheel liquid visualiser orienting mechanism with the direction of rotation of the eyeball and liquid indicated by arrows;
Figure 6 is an enlarged side elevation of the visualiser self-cleaning module of the eyeball of Figure 1 with the direction of rotation of the eyeball indicated by an arrow;
Figure 7(a) is a perspective view from above and one side of the scope having three internal channels in the probe made up of front and rear eyeball rotation channels and a data/cleaning channel and two external power channels for transmitting power to the eyeball;
Figure 7(b) is a side elevation of the scope of Figure 7(a);
Figure 8 is a side elevation of the scope of Figures 1 to 7(b) in which the probe is provided with a hinge to further assist with visualising the ear;
Figure 9 is an exploded view of the eyeball;
Figure 10 is a side view of another embodiment of the scope of the invention in which the image sensor is relocated from the eyeball into the probe and the eyeball is rotated to a 0° position;
Figure 11 is a side view of the scope of Figure 11 in which the image sensor is rotated to a 45° position;
Figure 12 is a side view of the scope of Figure 10 in which the image sensor is rotated to a 120° position;
Figure 13 is a side view of another embodiment of the invention similar to the embodiment of Figures 10 to 12 but with an alternative lens configuration rotated to a 0° position;
Figure 14 is a side view of the scope of Figure 13 in which the image sensor is rotated to a 45° position;
Figure 15 is a schematic representation of a further embodiment of the invention similar to the embodiments of Figures 10 to 14 in which the image sensors are stacked to save space and reduce the profile of the probe;
Figure 16(a) is a side elevation of a further embodiment of the scope in which the articulatable visualiser is a tiltable camera controllable with a directional lever and the scope is provided with a stabilizer in the form of a speculum;
Figure 16(b) is a side elevation of the endoscope of Figure 16(a) in which the directional lever is replaced by a directional twist knob;
Figure 17(a) is a side elevation of a further embodiment of the scope similar to the scope of Figures 16(a) and (b) but in which the probe is curved to conform with the speculum and provide free access space for a surgeon and the orientation of the camera is controllable via a directional wheel and the probe is slidable to adjust the depth of the probe;
Figure 17(b) is a side elevation of the endoscope of Figure 17(a) in which the orientation of the camera is adjustable via a slider;
Figures 18(a) to 18(b) are side elevations of a further embodiment similar to that of Figures 16 to 17 but in which the probe is also slidable with respect to the speculum;
Figure 19 is an enlarged cross-sectional view of a further embodiment of the invention in which the visualiser orienting mechanism is an external spring-operated living hinge to facilitate orientation of the camera;
Figure 20 is an enlarged cross-sectional view of an internal spring-operated living hinge of the probe orienting mechanism to further facilitate orientation of the camera;
Figure 21 is an enlarged cross-sectional view of the probe orienting mechanism in which a spring-operated double living hinge of the probe facilitates orientation of the camera;
Figure 22 is a side elevation of a further embodiment of the scope but in which the endoscope is further provided with an optical configuration that includes an angled prism adjacent the camera to assist in unobtrusive visualisation;
Figure 23 is an enlarged cross-sectional view through optical configurations of the probe showing the lens elements of the optical configuration which are movable to focus images;
Figure 24 is an enlarged cross-sectional view of an alternative optical configuration in which the optical configuration includes mechanically coupled elements;
Figure 25 is an enlarged cross-sectional view of yet an alternative optical configuration in which the optical configuration includes a solenoid and spring-operated staged focus arrangement;
Figure 26 is an enlarged perspective view from above and one side of the camera on the tip of a probe in which the scope is provided with four light emitters surrounding the camera;
Figure 27 is a schematic representation of various light emitter arrangements at the camera of the scope;
Figure 28 is a schematic representation of light being transmitted through the speculum to the probe camera;
Figure 29 is a side elevation of the scope provided with a double roller-driven visualiser orienting mechanism; and
Figure 30a and 30b are side elevational views of a visualiser self-cleaning module comprises an elastomeric sheath.

### Detailed Description of the Invention

All publications, patents, patent applications and other references mentioned herein are hereby incorporated by reference in their entireties for all purposes as if each individual publication, patent or patent application were specifically and individually indicated to be incorporated by reference and the content thereof recited in full.

### Definitions and general preferences

Where used herein and unless specifically indicated otherwise, the following terms are intended to have the following meanings in addition to any broader (or narrower) meanings the terms might enjoy in the art:
Unless otherwise required by context, the use herein of the singular is to be read to include the plural and vice versa. The term "a" or "an" used in relation to an entity is to be read to refer to one or more of that entity. As such, the terms "a" (or "an"), "one or more," and "at least one" are used interchangeably herein.

As used herein, the term "comprise," or variations thereof such as "comprises" or "comprising," are to be read to indicate the inclusion of any recited integer (e.g. a feature, element, characteristic, property, method/process step or limitation) or group of integers (e.g. features, element, characteristics, properties, method/process steps or limitations) but not the exclusion of any other integer or group of integers. Thus, as used herein the term "comprising" is inclusive or open-ended and does not exclude additional, unrecited integers or method/process steps.

As used herein, the term "disease" is used to define any abnormal condition that impairs physiological function and is associated with specific symptoms. The term is used broadly to encompass any disorder, illness, abnormality, pathology, sickness, condition or syndrome in which physiological function is impaired irrespective of the nature of the aetiology (or indeed whether the aetiological basis for the disease is established). It therefore encompasses conditions arising from infection, trauma, injury, surgery, radiological ablation, poisoning or nutritional deficiencies.

As used herein, the term "treatment" or "treating" refers to an intervention (e.g. the administration of an agent to a subject) which cures, ameliorates or lessens the symptoms of a disease or removes (or lessens the impact of) its cause(s). In this case, the term is used synonymously with the term "therapy".

Additionally, the terms "treatment" or "treating" refers to an intervention (e.g. the administration of an agent to a subject) which prevents or delays the onset or progression of a disease or reduces (or eradicates) its incidence within a treated population. In this case, the term treatment is used synonymously with the term "prophylaxis".

In the context of treatment and effective amounts as defined above, the term subject (which is to be read to include "individual", "animal", "patient" or "mammal" where context permits) defines any subject, particularly a mammalian subject, for whom treatment is indicated. Mammalian subjects include, but are not limited to, humans, domestic animals, farm animals, zoo animals, sport animals, pet animals such as dogs, cats, guinea pigs, rabbits, rats, mice, horses, cattle, cows; primates such as apes, monkeys, orangutans, and chimpanzees; canids such as dogs and wolves; felids such as cats, lions, and tigers; equids such as horses, donkeys, and zebras; food animals such as cows, pigs, and sheep; ungulates such as deer and giraffes; and rodents such as mice, rats, hamsters and guinea pigs. In preferred embodiments, the subject is a human.

### Exemplification

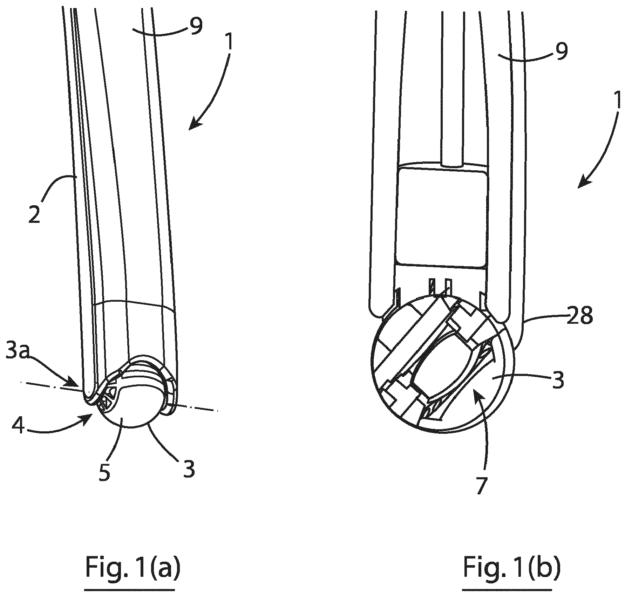

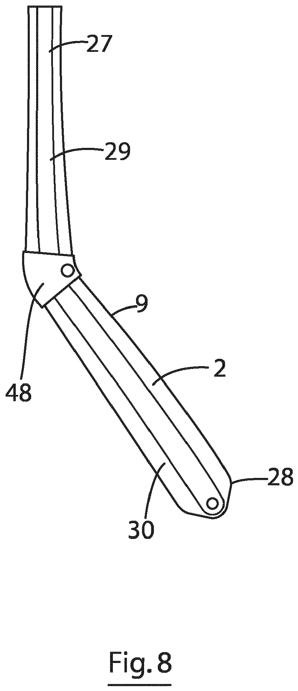

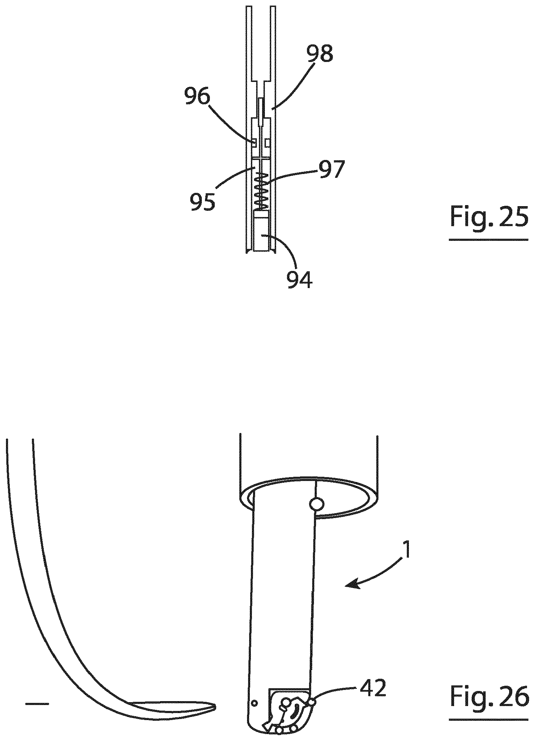

As shown in Figures 1 to 9 of the accompanying drawings, a scope of the invention is generally indicated by the reference numeral 1 and is made up of a probe 2 and an articulatable visualiser 3 which can be oriented/tilted and/or rotated about a visualiser axis 3a via an orienting mechanism 4 for optimal viewing of the ear. In the present embodiment, the articulatable visualiser 3 is a substantially spherical rotatable eyeball-like image sensor or camera 5. However, as will be appreciated by those skilled in the art, in other embodiments, the articulatable visualiser 3 can have other non-spherical eyeball shapes provided the articulatable visualiser has a uniform profile about its articulation or rotational axis 3a. The scope 1 is also provided with a light source 42 which can be incorporated into the camera 5 as in the present embodiment or separate from/adjacent to the camera 5 as shown for example in Figures 26 to 28. The scope can be an otoscope for examining ears or a surgical endoscope which allows for bi-manual diagnosis and surgical techniques whilst benefiting from the advantages associated with endoscopic visualisation of the ear.

The scope 1 is further provided with a self-cleaning module 6 for cleaning detritus from the eyeball camera while in use without requiring removal of the scope from the ear. This shall be explained more fully below. The eyeball camera 5 is also fitted with an optical configuration 7 to optimise images from the eyeball camera 5 made up of lens elements 8 and a focusing mechanism 41 for controlling the lens elements.

The probe 2 is made up of an elongate tubular body 9 defining an open mouth 10 at its distal end for holding the eyeball camera 5. Externally, the elongate tubular body 9 is provided with first and second oppositely disposed side channels 11,12 respectively for receiving respective detachable first and second side panels 13,14. The first and/or second side channels 11,12 serve to house power lines 15 for powering the eyeball camera 5 and terminate at power pins 16 insertable in the eyeball camera 5.

At the mouth 10, the probe tubular body 9 is shaped to define two oppositely disposed axis holes 17 for supporting the eyeball camera 5 in the mouth 10 at two oppositely disposed axis pins 20, which define the visualiser axis 3a and extend laterally outwards from the eyeball camera 5.

A data cable 18 extends through the tubular body 9 from the eyeball cameral 5 via a wireless communicator module 19.

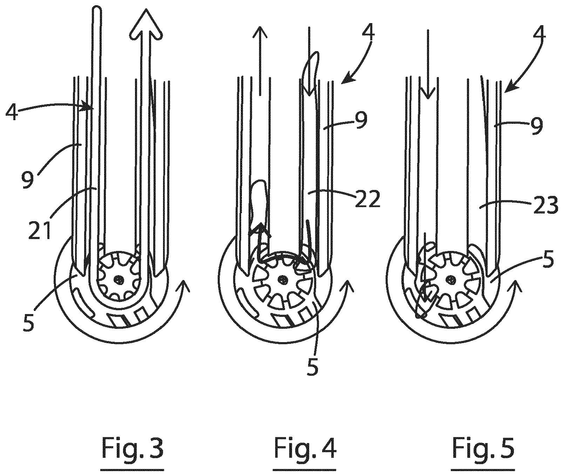

The orienting mechanism 4 for rotating the eyeball camera 5 in the mouth 10 of the probe 2 can function in a number of ways as desired. For example, as shown in Figure 3, the orienting mechanism 4 can be a belt- or band- driven orienting mechanism 21 while, as shown in Figure 4, the orienting mechanism 4 can be a hydraulic orienting mechanism 22 operating similar to a vane or gear pump. Alternatively, as shown in Figure 5, the orienting mechanism 4 can be an air orienting mechanism 23 operating in a manner similar to a centrifugal pump or pelton wheel.

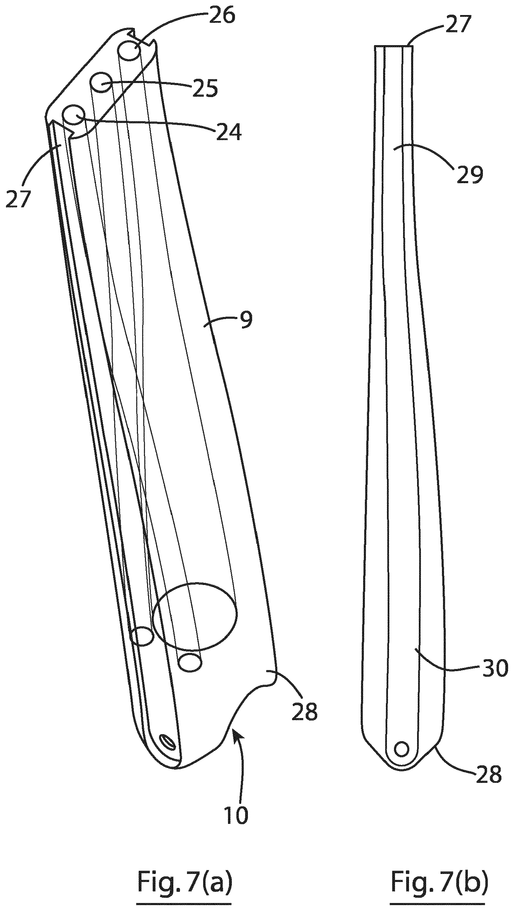

As shown particularly in Figure 7(a), the orienting mechanisms 4 of Figures 3 to 5 can be accommodated in first and second internal channels 24,25 defined in the probe elongate body 9. In addition, the probe elongate body 9 can be provided with an internal data and/or cleaning channel 26 for receiving the data cable 18 and/or carrying fluid and detritus to and/or from the self-cleaning module 6. The data channel 26 is large at the distal end 28 where it holds the wireless communication module 19 and cleaning module 6 while the first and second internal channels 24,25 are stacked at the distal end 28. Proximally from the distal end 28 the first and second internal channels 24,25 reorientate and resize as shown in 7(a) to allow for a more narrow profile probe towards the proximal end 27 as indicated by the reference numeral 29 than towards the distal end 28 as indicated by the reference numeral 30, thus making it easier to manoeuvre tools past the probe as shown in Figure 7(b). Moreover, the resultant narrow profile 29 allows for flexible bending of the probe 2 at a hinge 48.

In other embodiments, additional internal channels can be provided to supply liquid air and vacuum to integrate with the cleaning module 6 and for use in surgery. Alternatively or in addition, a working channel can be provided for tools such as a needle for injection.

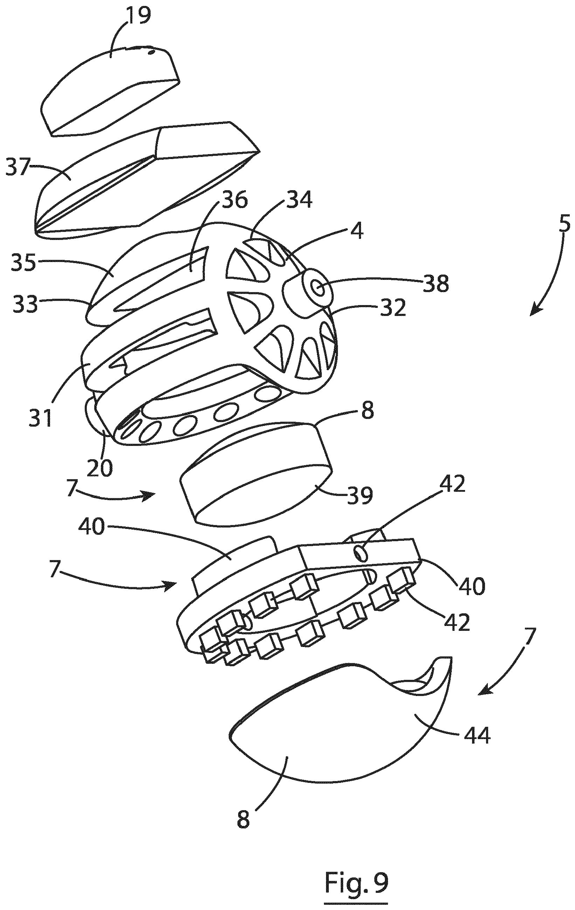

As shown particularly in Figure 9, the eyeball camera 5 of the present embodiment is made up of a rotatable cage-like housing 31 having a first side 32 and an opposite second side 33. The housing 31 is provided with an element of the orienting mechanism 4 in the form of a wheel 34 formed at the second side 33 of the housing 31. The first side 32 is provided with a first axis pin 20 as previously described while the wheel is provided with a second axis pin 20 defining the rotational axis 3a. The wheel 34 is operable by the orienting mechanism 4 to rotate the housing 31 on the axis pins 20 and hence the eyeball camera 5. The axis pins 20 each have power sockets 38 for receiving power from the power pins 16. The cage-like housing 31 is further provided with ribs 35 extending between the first and second sides 32,33 which define cage board slots 36 therebetween for receiving and holding functional boards of the eyeball camera 5 such as an image sensor board 37.

The eyeball camera 5 has an internal focusing lens 39 forming part of the lens elements 8 of the optical configuration 7 together with a main board 40 provided with a focus mechanism which also forms part of the optical configuration 7. The eyeball camera 5, and in the present embodiment the main board 40, also has a light source 42 in the form of light emitters 42 on an opposite face thereof for illuminating the ear and oppositely disposed power pin contacts 43 for receiving power at the power sockets 38. The eyeball camera 5 also has an external curved front lens 44 also forming part of the lens elements 8 of the optical configuration 7.

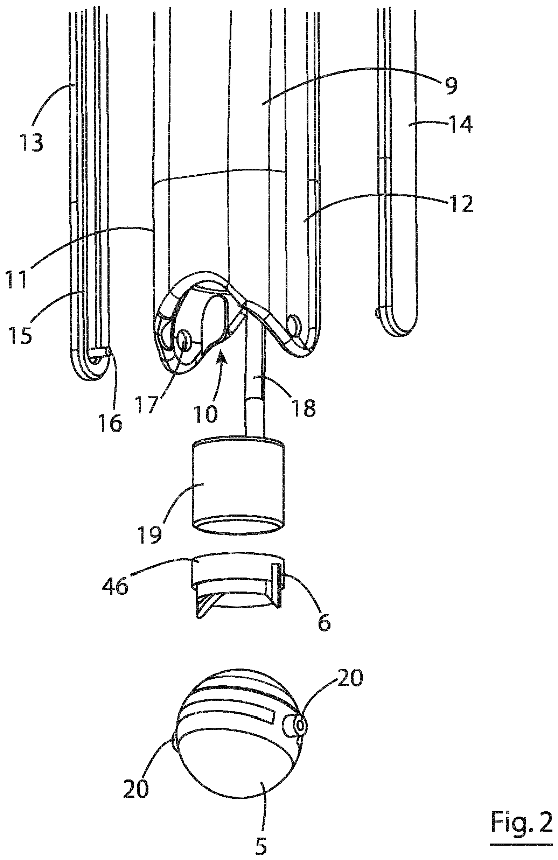

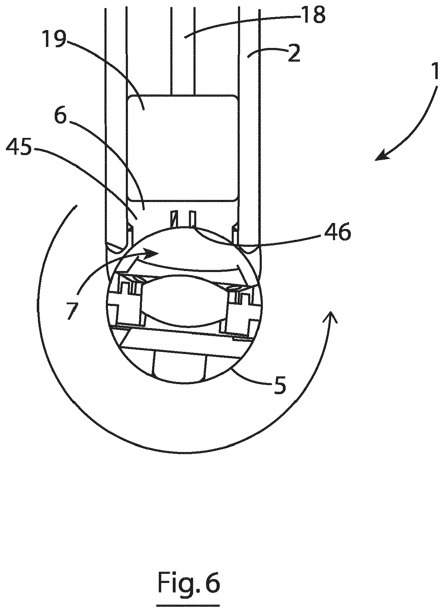

The self-cleaning module 6 (see Figures 2 and 6) is made up of a ring-like body 45 provided with cleaning blades 46 and a soft cleaning material 47 all shaped and contoured to clean the curved front lens 44 of the eyeball camera 5.

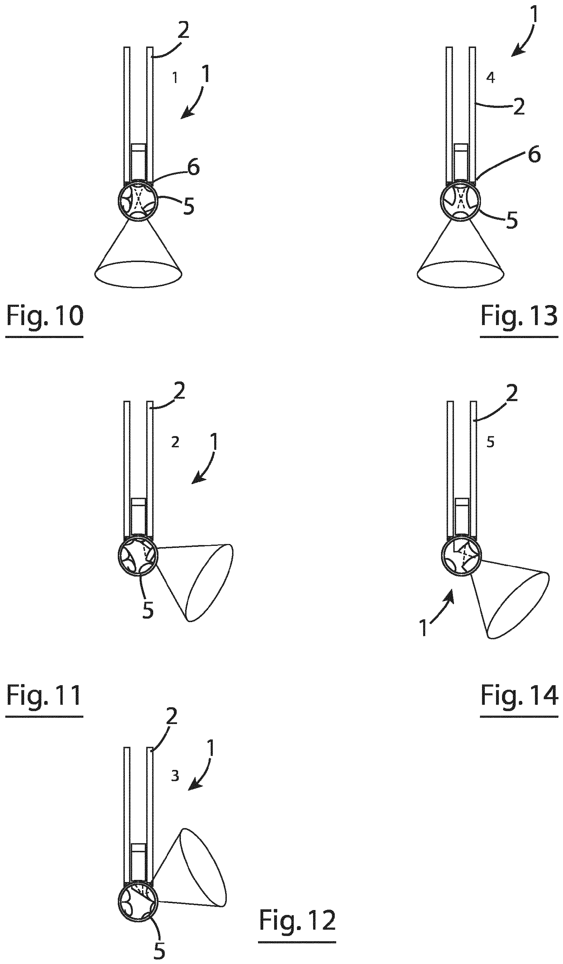

Figures 10 to 14 show another embodiment of the scope 1 of the invention in which the image sensor board 37 is relocated from the eyeball camera 5 as shown in Figures 1 to 9 into the probe body 9. The eyeball camera 5 can be rotated as shown in the drawings while the self-cleaning module 6 serves to clean the eyeball camera 5 as previously described.

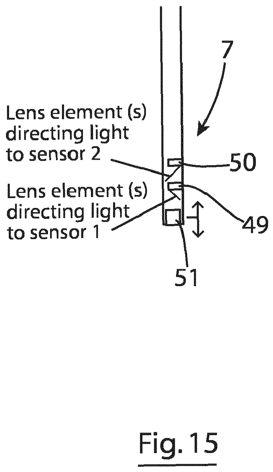

Figure 15 shows a schematic representation of a further embodiment of the invention in which the image sensor boards 37 are stacked to save space and reduce the profile of the probe 1 so that the scope 1 can fit into different lumens in the body. As the image sensors are stacked to save space and the lenses deliver a spatially separated image to each sensor, image processing software can deliver this image in a binocular view to give depth perception with a low-profile probe. More particularly, as shown in the drawing, the stacked optical configuration 7 is made up of first and second stacked fixed image sensors 49,50 and corresponding first and second lens elements 8 directing light to the image sensors 49,50 together with a slidably adjustable front lens 51.

In one embodiment aspherical lenses can be used to bypass the first fixed sensor 49 in an efficient manner e.g. crescent profile lenses (in plan view).

In use, the eyeball camera 5 of Figures 1 to 9 is held in the probe 2 by the axis pins 20 which are held in the axis holes 17. This allows the eyeball camera 5 to rotate fully 360° on the axis 3a - the rotation has the dual purpose of reorienting the camera direction and cleaning the front lens 44.

Accordingly, the front lens 44 is cleaned by rotating 360°, as shown in Figure 6, thus wiping the front lens 44 with the self-cleaning module 6 which is held in the probe 2. The self-cleaning module 6 consists of materials to clean the lens such as the flexible blade 46 and soft material 47. In another variant of the invention, the cleaning module 6 can be integrated with internal vacuum and liquid channels in the probe 2.

Because the eyeball camera 5 is only connected to the probe at the axis pins 20, there is limited space for power and data pins/contacts or cables especially in smaller size implementations. Accordingly, only one power pin contact 43 is delivered through each axis pin 20 so that power is delivered through these pin contacts 43 and data from the image sensor can be delivered using a wireless technology between the wireless communicator 19 from which the data cable 18 delivers data to an image processor outside of the scope 1.

The wireless communicator 19 of the eyeball camera 5 can also measure the angle of rotation which can be displayed to the user. In another embodiment, data can also be delivered by signal modulation in the two power pins 16. In this case, an image processor must separate the data signal from the power to display an image.

As shown in Figures 3 to 5, the eyeball camera 5 can be controlled using a belt or band orienting mechanism 21, a hydraulic orienting mechanism 22 or a liquid orienting mechanism 23 in a fashion similar to a vane or gear pump. Fine controls for systems using hydraulic actuation can be provided through manual push buttons on the scope 1. In other embodiments, the orienting mechanism can be a motor built into the probe 2 or the eyeball camera 5. Alternatively, a wheel or gear in the probe 2 actuates the eyeball camera 5 or a wire can be used in a crank fashion to rotate the eyeball camera 5.

In other embodiments of the invention, there are a different number of electrical boards and the functions are located between them.

The internal focus lens 39 is held in position by the focusing mechanism 41 of the optical configuration 7. The focusing mechanism 41 positions the lens between the front lens 44 and the image sensor board 37 and repositions the focus lens 39 to change focus of the eyeball camera 5. The focusing mechanism 41 is electromechanically operated.

In one variant, the focusing mechanism 41 can employ electromagnets and in other embodiments, the focusing mechanism 41 can employ bimetal linkages. Alternatively, harmonics can be used to control linkages which move the focusing lens 39.

In another embodiment of the invention, the optical configuration can be made up of a variable aperture which can be employed instead of a focusing mechanism 41 deploying lens elements 8 moving relevant to or in conjunction with the image sensor. The aperture may be electromechanical in nature or digital where an opaque filter forms the aperture. Change of the aperture size allows change in the depth of field which can be advantageous because a large depth of field (small aperture) can be used to observe tools from a distance. The depth of field can then be reduced by expanding the aperture, to allow high quality imaging at close range.

The lens elements 8 can be made up of multiple elements to achieve best image quality while, in some embodiments, another variation the outer layer of the front lens 44 is a window to allow in light with minimum distortion.

In some embodiments, flexible optics are used to change the surface profile of the lens elements 7 to change the focus.

If desired, additive manufacturing can be used to 3d print the lenses.

As indicated above, in some embodiments, the articulatable visualiser is not spherical but can be configured differently e.g. a cylindrical or other uniform profiles around its axis 3a. Uniform profiles such as a cylinder or sphere are advantageous in use because the uniform profile doesn't change shape when rotated against tissue and is less likely to cause trauma to tissue. In another embodiment, a window also surrounds the eyeball camera 5 with a profile similar to a U to ensure that the eyeball camera 5 does not cause trauma to tissue.

However, in other embodiments, the profile of the articulatable visualiser 3 need not be uniform around its axis 3a.

In another embodiment, the front lens 44 protrudes further than the rest of the eyeball camera 5, to contact with the self-cleaning module 6 when rotated, while the rest of the eyeball camera 5 does not contact the self-cleaning module.

If desired, the scope of the invention can include two cameras 5 to give binocular vision while in other embodiments a single camera 5 uses two lenses to project onto a single sensor to give binocular vision, using out of phase images at full resolution and a filter built into each lens turns opaque at the same frequency to separate the two images.

In another embodiment, two lenses are projected onto a single sensor to give binocular vision, lens-lets on the image sensor are tuned to either one lens or the other based on light entry angle, approximately 50% tuned to one lens and approximately 50% to the other and image processing techniques separate the image to give binocular vision, although with reduced resolution.

Figures 10 to 12 show an eyeball camera 5 in which the image sensor is relocated from the eyeball into the probe which can operate at 0° through the centre, 45° using a lens to one side, and 120° through another side using lenses and prism(s). Figures 13 and 14 show another configuration with 0° through the centre and with 45° also through the centre. In one embodiment, optical filters are applied to the lens to prevent light from the wrong lens entering the sensor. If desired, light emitters 42 can be built into the eyeball camera 5 as previously described or, alternatively, the light emitters 42 can be located on the probe 2.

Figure 15 shows a schematic representation of a further embodiment of the invention in which the image sensor boards 37 are stacked in the probe 2 to save space and reduce the profile of the probe 2 so that the scope 1 can fit into different lumens in the body. As the image sensors are stacked to save space and the lenses deliver a spatially separated image to each sensor, image processing software can deliver this image in a binocular view to give depth perception with a low-profile probe 2. More particularly, as shown in the drawing, the stacked optical configuration 7 is made up of first and second stacked fixed image sensors 49,50 and corresponding first and second lens elements 8 directing light to the image sensors 49,50 together with a slidably adjustable front lens 51.

In one embodiment aspherical lenses can be used to bypass the first fixed sensor 49 in an efficient manner e.g. crescent profile lenses (in plan view).

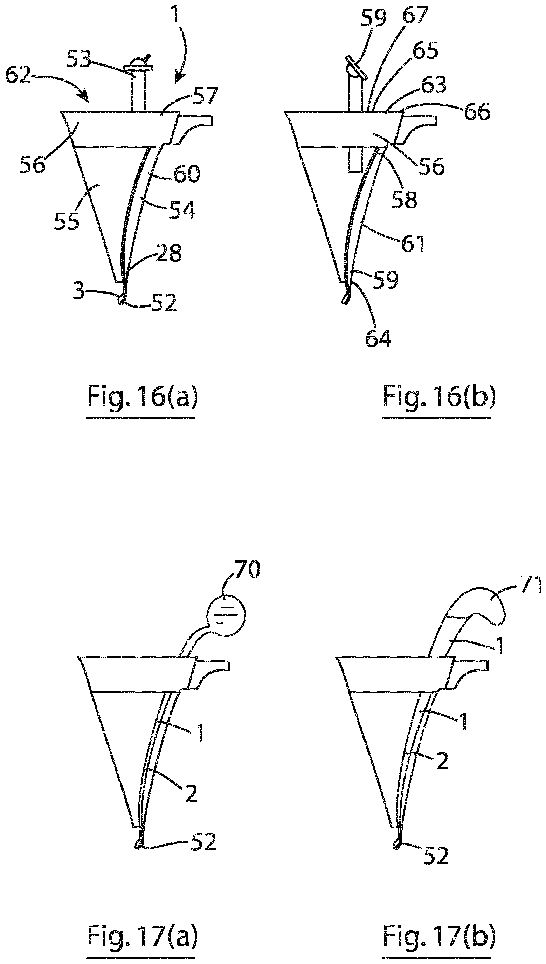

Figure 16(a) shows a side elevation of a further embodiment of the scope 1 in which the articulatable visualiser 3 is a tiltable camera 52 controllable with an orienting mechanism 4 in the form of a directional lever 53 and the scope 1 is provided with a stabilizer in the form of a speculum 55. More particularly, the speculum 55 is attached to a speculum holder in the form of a speculum handle 56. The speculum handle 56 has a collar 57 and the probe 2 is attached to the collar 57 and located in the speculum 55 with the tiltable camera 52 at the distal end 28 of the probe 2. The probe 2, being integrated with the speculum holder 56 in a unitary structure, defines a unified speculum-probe scope assembly. As previously described, the scope assembly can be in the form of an otoscope for examining ears or a surgical endoscope which allows for bi-manual diagnosis and surgical techniques whilst benefiting from the advantages associated with endoscopic visualisation of the ear.

The speculum 55 is made up of a proximal open end 58 through which a surgeon can access an ear during surgical procedures and a distal insertion end 59 insertable in an ear. A substantially conical speculum wall 60 extends between the proximal and distal ends 58,59 which defines an internal chamber 61 for receiving the probe 2 and surgical instruments in use. The conical speculum wall 60 further defines a relatively large surgical access opening 62 at a rim 63 at the proximal end 58 and a relatively narrow insertion opening 64 at the distal end 59 through which a surgeon can access the ear during surgical procedures.

The collar 57 of the handle 56 has speculum mounting in the form of a ring 65 defining a bore 66 for receiving the rim 63 of the speculum to mount and secure the handle 56 to the rim 63 of the speculum 55. The collar 57 is further provided with a probe mounting 67 to mount the probe 2 on the handle so that the elongate probe 2 can extend through the speculum from the proximal end 58 to the distal end 59 and exit the distal end 59 through the insertion opening 64 if required.

As shown in the drawing, the orienting or tilting mechanism 4 can be employed to orient the tiltable camera 52 as required. The orienting mechanism 4 is manually controllable with the probe direction lever 53.

Figure 16(b) is a side elevation of a scope 1 similar to the scope 1 of Figure 16(a) but in which the probe direction lever is in the form of a manually operable probe knob 69.

Figure 17(a) is a side elevation of a fourth embodiment of the scope 1 similar to the scope 1 of Figures 16(a) and 16(b) but in which, in addition to the elongate probe 2 being slidable and orientable, the elongate probe 2 is curved to conform with the wall 60 of the speculum 55 to create additional space in the speculum chamber 61 for a surgeon in use. The scope 1 is provided with a probe direction wheel 70 in place of the probe direction lever of Figure 16(a).

Figure 17(b) is a side elevation of the endoscope of Figure 17(a) but in which the probe direction wheel 70 is replaced by a probe direction slider 71.

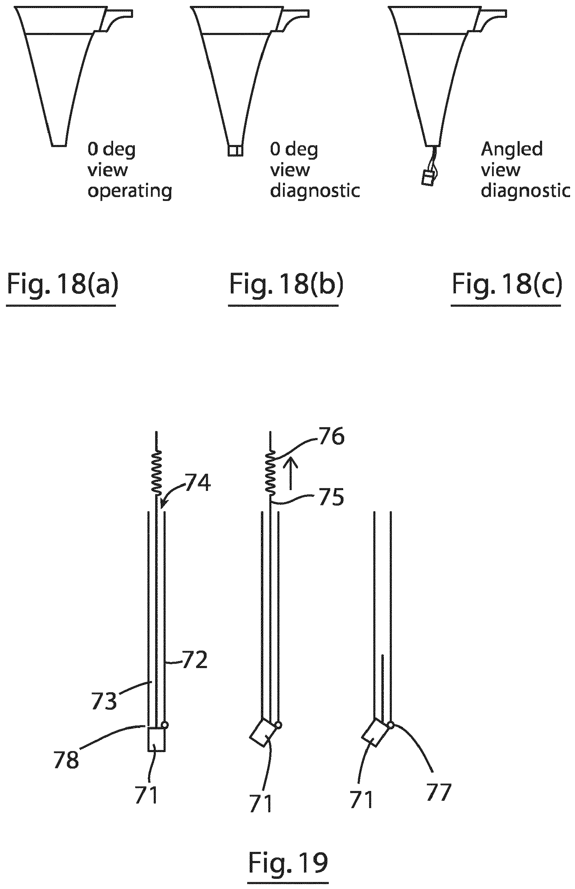

Figures 18(a) to 18(c) show side elevations of an embodiment of the invention similar to that of Figures 16 and 17 but in which the probe 2 is also slidable with respect to the speculum 2.

Figure 19 is an enlarged cross-sectional view of a portion of the orienting mechanism 4 for the tiltable camera 52. As shown in the drawing, the probe orienting mechanism is made up of the titlable camera 52 including a light source in the form of a camera module 71 at the distal end of the elongate probe 2 which is made up of an outer shaft 72, an inner shaft 73 within the outer shaft 72 and a channel 74 for housing a actuating cable 75 which extends between the camera module 71 and a spring 76 (or other elastic material) of the orienting mechanism 4. The camera module 71 is mounted on the outer shaft 72 at an external living hinge 77 with a tilt recess 78 disposed opposite the external living hinge 77 so that linear movement of the inner shaft 73 relative to the outer shaft 72 causes the camera module 71 to tilt.

The living hinge 77 therefore retains the camera module 71 so that it tilts in a predictable way. In other embodiments of the invention, the living hinge 77 can be replaced by other locators such as a magnet or ball and socket. In still further embodiments, the data cable 18 can double as the actuator cable 75.

The portion of the orienting mechanism 4 of Figure 19 can also be provided with a protective elastic cover to protect subjects from sharp edges.

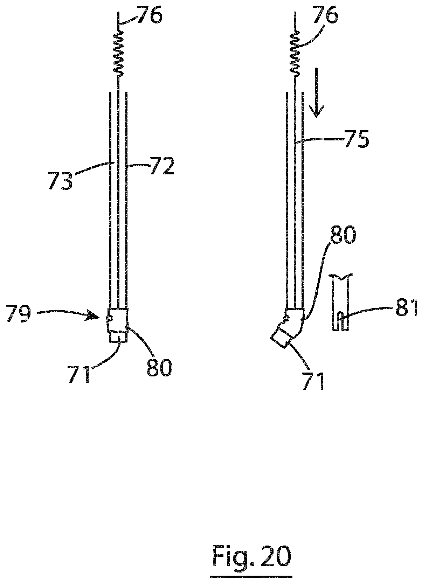

Figure 20 shows an enlarged cross-sectional view of a portion of the probe orienting mechanism 4 similar to the probe orienting mechanism 4 of Figure 19 (like numerals indicate like parts) but in which the external living hinge 77 is replaced by an internal spring-operated living hinge 79 to facilitates orientation of the camera module 71 by rotary motion of the inner shaft 73 relative to the outer shaft 72. As shown in the drawing, the internal living hinge 79 is provided with a hinge cover 80 and is located at an inner shaft slot 81 so that rotation of the inner shaft 73 causes the camera module 71 to rotate as shown in the drawing.

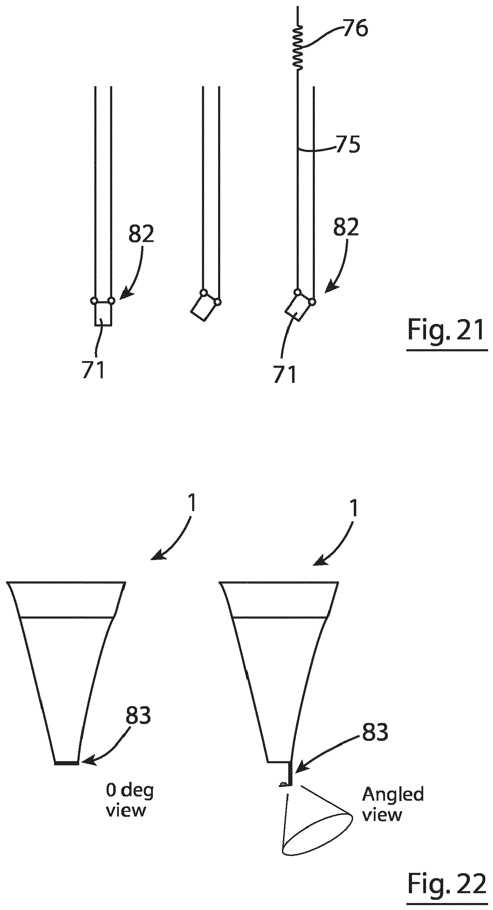

Figure 21 is an enlarged cross-sectional view of a portion of the orienting mechanism 4 in which a spring-operated double living hinge 82 of the probe facilitates orientation of the camera probe 2. In this embodiment, the outer shaft 72 is fully or partially split allowing the inner and outer sides to move relative to each other.

Figure 22 shows side elevations of a further embodiment of the scope 1 similar to the scope of Figures 16 to 21 but in which the scope 1 is further provided with an angled prism 83 to assist in unobtrusive visualisation.

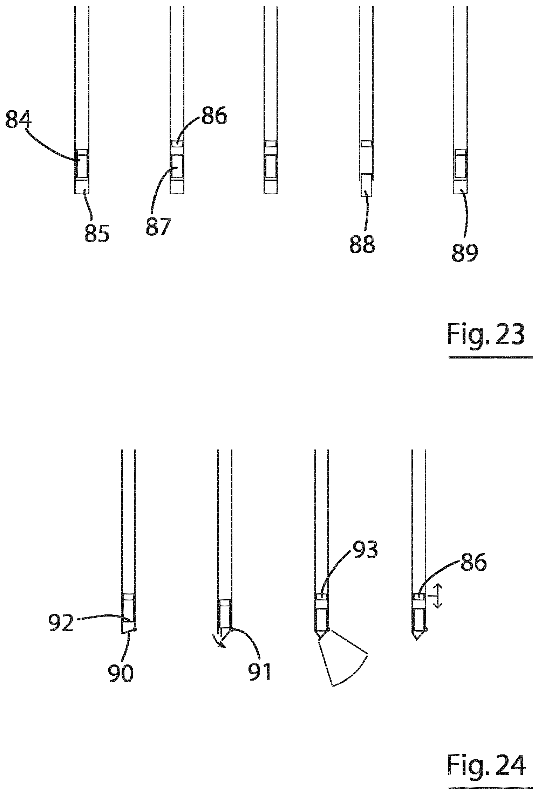

Figure 23 shows enlarged cross-sectional views of various optical configurations 7 of showing the lens elements 8 of the optical configurations 7 which are movable to focus images. More particularly, the optical configurations 7 can be made up of combinations of an adjustable lens and sensor element 84, a fixed front element 85, and adjustable sensor 86, a rod lens 87, an adjustable front lens 88 and an angled front lens 89.

Figure 24 shows enlarged cross-sectional views of an alternative optical configuration 7 in which the optical configuration 7 includes a tilting front lens 90 attached to the elongate probe 2 at a lens hinge 91 and mechanically coupled via a mechanical coupling 92 to another element so that as the front lens 90 is tilted, the coupled element moves to ensure image transmission. The optical configuration can be further provided with a fixed sensor 93. Additional moving elements can also be included to adjust the focus such as an adjustable sensor 86.

Figure 25 shows an enlarged cross-sectional view of yet an alternative optical configuration 7 in which the optical configuration 7 is a staged focus optical configuration 7. In the present embodiment, the optical configuration is provided with a lens and/or sensor moving element 94 and a front end stop 95 spaced apart from a rear end stop 96 to determine the position of the lens and sensor moving element. The optical configuration 7 has two focus positions. In one position, the lens and/or sensor moving element 94 is pulled back fully to rest at the first predetermined focus position (the rear end stop 96). In the second position, the lens and/or sensor moving element 94 is pushed fully forward to the second focus position (front end stop 95). The small amount of movement required (potentially under 0.1mm) to adjust the focus in the required range is achieved with a mechanism consisting of a spring 97 and a solenoid 98. In an alternative embodiment, a spring and actuator under a ratchet mechanism (similar to a click pen) or a friction controlled mechanism can be employed.

The two focus positions firstly allow the surgeon look in detail at the anatomy and secondly allow the camera to be pulled back to view the tools in the surgical field during operations.

Figure 26 shows a further embodiment of the invention in which the probe 2 is provided with four light emitters 42 surrounding the articulatable visualiser 3.

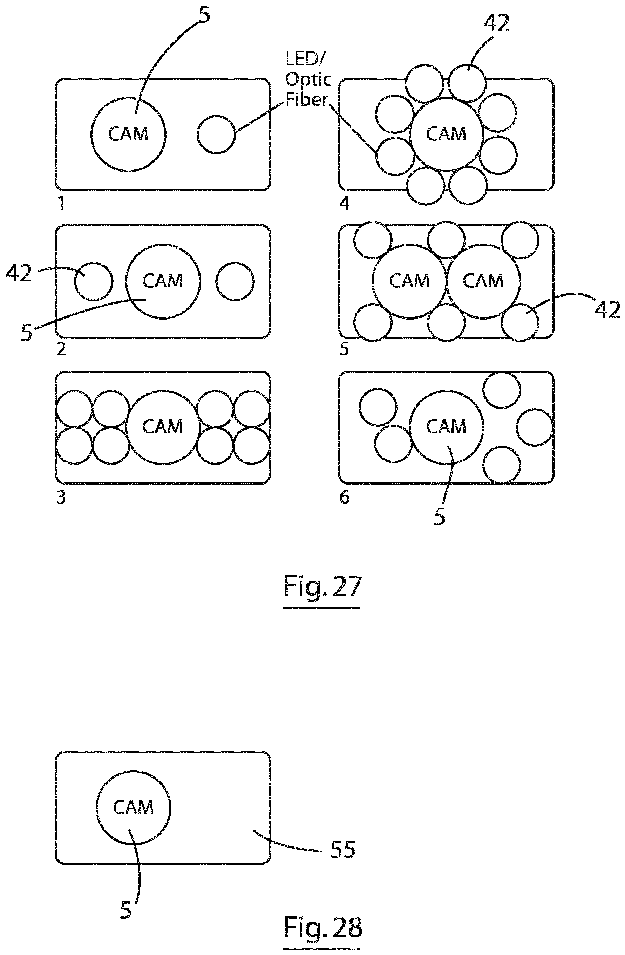

Figure 27 shows a schematic representation of various light emitter 42 arrangements. Light is delivered through the probe 1 from a light source at the proximal end 27 using for example optic fibers which can be one large optic fiber or multiple optic filters in different formats, including but not limited to those shown in the drawing. In addition, the distal surface of the optic fiber can be planar or curved to allow light dispersion.

As shown in Figure 28, light can also be transmitted through the structure of the scope 1 e.g. through the speculum 55 to the articulatable visualizer 3.
The scope 1 and systems of the invention can be formed from any suitable materials e.g. biodegradable materials while additive manufacturing is utilized to make the lenses and other components of the copes of the invention.

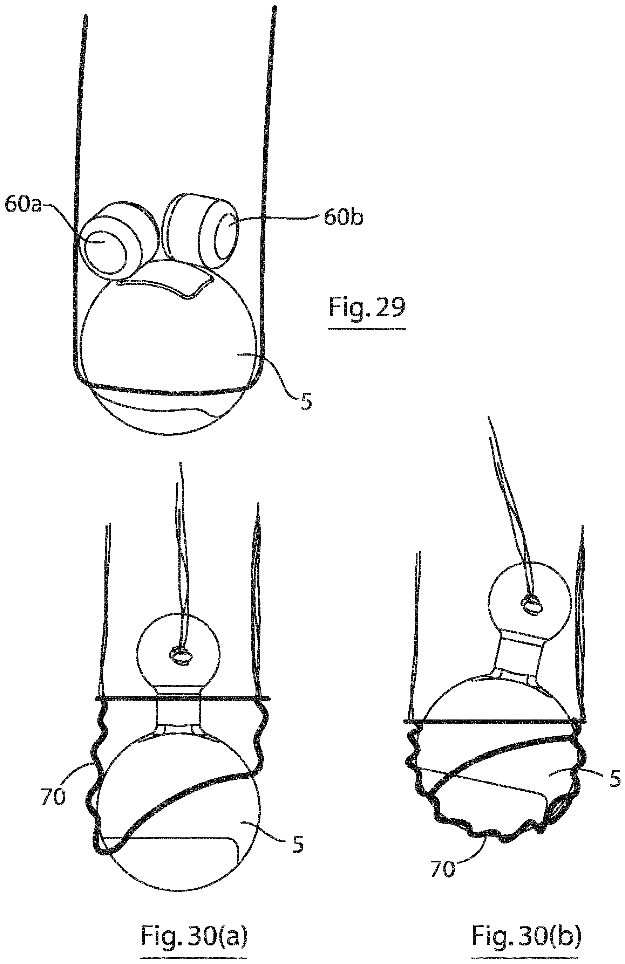

Referring to Figure 29, an embodiment of the scope of the invention is described in which parts described with reference to previous embodiments are assigned the same reference numerals. In this embodiment, the orientating mechanism for the visualizer 5 comprises a pair of rollers 60a, 60b in contact with the visualizer 5 and configured for rotation about axes which are generally orthogonal to each other. It will be appreciated that the two rollers can be employed in combination to effect rotation of the visualiser about an infinite number of axes.

Referring to Figure 30, an embodiment of the scope of the invention is described in which parts described with reference to previous embodiments are assigned the same reference numerals. In this embodiment, the self-cleaning module comprises an elastomeric sheath 70 configured for movement distally from a retracted position shown in Fig. 30a to a deployed position shown in Fig. 30b where the sheath closes over the lens of the visualizer 5 wiping it clean.

### Equivalents

The foregoing description details presently preferred embodiments of the present invention. Numerous modifications and variations in practice thereof are expected to occur to those skilled in the art upon consideration of these descriptions. Those modifications and variations are intended to be encompassed within the claims appended hereto.

## Claims

1. A scope for examining or surgically treating an internal structure of the ear comprising:
a probe;
at least one visualiser on the probe having an optical configuration, and
a light source
wherein the visualiser is articulatable relative to the probe by an orienting mechanism for optimal viewing of the internal structure of the ear.

2. A scope as claimed in Claim 1 wherein the visualizer comprises a communication module configured for wirelessly transmitting or receiving data with a communication module in the probe, optionally the probe is configured to wirelessly transmit power to the visualizer.

3. A scope as claimed in Claim 1 or 2 wherein the visualiser is rotatable about a visualiser articulation axis.

4. A scope as claimed in Claim 3 wherein the visualiser has a uniform profile about its articulation axis.

5. A scope as claimed in any preceding Claim wherein the visualiser is substantially spherical and in which the probe optionally comprises a socket for receipt of the substantially spherical visualizer for rotation of the visualiser about multiple axes.

6. A scope as claimed in any of Claims 1 to 5 wherein the visualiser comprises an image sensor and a lens and is optionally configured to focus the visualizer by adjustment of the distance between the lens and image sensor.

7. A scope as claimed in Claim 6, in which the visualizer is adjustable between two or more pre-set focus settings.

8. A scope as claimed in any of Claims 1 to 8 wherein the probe exhibits a nonuniform profile, and includes a narrowed profile at a proximal end or a centre part.

9. A scope as claimed in any preceding Claim wherein the orienting mechanism is a belt- or band- driven orienting mechanisma fluidic orienting mechanism, or driven using magnetic forces such as a motor, directly acting on the visualiser, or acting on a wheel or linkage in communication with the visualiser.

10. A scope as claimed in any preceding Claim further comprising a self-cleaning module for cleaning detritus from the visualiser.

11. A scope as claimed in Claim 10 wherein the self-cleaning module comprises a cleaning surface and/or a fluid dispenser.

12. A scope as claimed in Claim 11 wherein the self-cleaning module is configured to provide relative movement between the visualizer and the cleaning surface to clean the visualizer.

13. A scope as claimed in any of Claims 1 to 12 further comprising a stabilizer for supporting the scope.

14. A scope as claimed in Claim 13 wherein the stabilizer comprises a speculum.

15. A scope as claimed in any of Claims 1 to 14 wherein the scope is an endoscope or otoscope.
